# EUROPÄISCHE PATENTSCHRIFT

(11) **EP 3 114 480 B1**
(45) Veröffentlichungstag und Bekanntmachung des Hinweises auf die Patenterteilung: **11.10.2023**
(21) Anmeldenummer: 15708806.3
(22) Anmeldetag: 06.03.2015
(51) Int. Cl.: G01N 21/65, G01N 33/49, G01N 33/50, G01N 33/80

(54) **VERFAHREN UND VORRICHTUNG ZUR QUALITÄTSKONTROLLE EINES BLUTPRODUKTS**
METHOD AND DEVICE FOR QUALITY CONTROLLING A BLOOD-BASED PRODUCT
PROCÉDÉ ET DISPOSITIF POUR CONTRÔLER LA QUALITÉ D'UN PRODUIT À BASE DE SANG

(30) Priorität: 07.03.2014 DE 102014003386
(43) Veröffentlichungstag der Anmeldung: 11.01.2017
(73) Patentinhaber: microPhotonX GmbH, 82327 Tutzing (DE)
(72) Erfinder: SCHÜTZE, Raimund, 82327 Tutzing (DE); SCHÜTZE, Karin, 82327 Tutzing (DE)
(74) Vertreter: Banzer, Hans-Jörg
(86) Internationale Anmeldenummer: PCT/EP2015/054724
(87) Internationale Veröffentlichungsnummer: WO 2015/132384

(56) Entgegenhaltungen:
- WO-A1-2010/048678
- WO-A1-2014/007759
- WO-A2-2004/008121
- WO-A2-2006/130728
- WO-A2-2008/027942
- CN-A- 1 242 937
- DE-A1-102008 060 332
- DE-T2- 69 734 363
- US-A1- 2010 136 609
- KARIN SCHÜTZE ET AL: "LASER World of PHOTONICS - DGLM Application Panel Unmet Needs in Photonics and Medicine: Novel cell analysis based on Raman spectroscopy", PHOTON LASERS MED, Bd. 2, 1. Januar 2013 (2013-01-01), Seiten 361-369, XP055191970, DOI: 10.1515/plm-2013-0043
- S. KOCH ET AL: "Novel cell identification: markerfree and suitable for living cells", PROCEEDINGS OF SPIE, Bd. 8798, 18. Juni 2013 (2013-06-18), Seite 87980J, XP055191973, ISSN: 0277-786X, DOI: 10.1117/12.2033542
- Jürgen Luhm ET AL: "Potential use of Raman Spectroscopy in the Quality Control of blood products", , 1. November 2013 (2013-11-01), Seiten 1-1, XP055191953, Gefunden im Internet: URL:http://celltool.de/files/201311_luhm-d rk_raman_red.pdf [gefunden am 2015-05-28]
- Raktim Dasgupta ET AL: "Studies on erythrocytes in malaria infected blood sample with Raman optical tweezers", International Society for Optical Engineering, vol. 16, no. 7, 1 July 2011 (2011-07-01), pages 077009-1, XP055463836, PO Box 10 Bellingham WA 98227-0010 USA ISSN: 1083-3668, DOI: 10.1117/1.3600011
- Myung-Hyun Nam ET AL: "Evaluation of Plasmodium vivax ELISA for the blood screen : Plasmodium vivax ELISA for blood screen", TROPICAL MEDICINE AND INTERNATIONAL HEALTH., vol. 15, no. 12, 1 December 2010 (2010-12-01), pages 1436-1441, XP055463841, GB ISSN: 1360-2276, DOI: 10.1111/j.1365-3156.2010.02657.x

## Beschreibung

### GEBIET DER ERFINDUNG

Die Erfindung betrifft Verfahren und Vorrichtungen zur Qualitätskontrolle eines Blutprodukts, mit denen bestimmbar ist, ob ein Erythrozytenkonzentrat, ein Thrombozytenkonzentrat, ein Granulozytenkonzentrat, ein Leukozytenkonzentrat, eine Konserve mit Vollblut und/oder eine Konserve mit Blutplasma noch zur Transfusion verwendet werden darf.

### HINTERGRUND DER ERFINDUNG

Blutprodukte, wie beispielsweise Erythrozytenkonzentrate, Thrombozytenkonzentrate, Granulozytenkonzentrate, Leukozytenkonzentrate, Vollblutkonserven oder Blutplasmakonserven, werden heute in Form von Blutkonserven in großem Umfang in Krankenhäusern verbraucht. Die Gewinnung der Konzentrate von humanen Blutspendern zur Transfusion ist ein aufwendiges Verfahren. Unter idealen Lagerbedingungen bei einer Temperatur zwischen 2°C und 6°C beträgt die Haltbarkeit der Konserven mit einem Erythrozytenkonzentrat beispielsweise 42 Tage. Thrombozytenkonserven müssen bislang typischerweise bereits nach einem kürzeren Zeitraum verworfen werden.

Die Qualitätskontrolle von Blutprodukten erfolgt häufig durch entsprechende Bestätigungen von Fachpersonal, dass eine Kühlkette eingehalten wurde. Da dennoch eine Unterbrechung der Kühlkette in vielen Fällen nicht ausgeschlossen werden kann, die die Haltbarkeitsdauer wesentlich verkürzen kann, werden Konserven, bei denen nicht sicher festgestellt werden kann, dass die Kühlkette ununterbrochen eingehalten wurde, möglicherweise bereits vor Erreichen des Verfallsdatums aus Sicherheitsgründen ebenfalls verworfen. Auch kann es sein, dass Blutspender Krankheitskeime oder negative Faktoren tragen, die zum Zeitpunkt der Blutspende nicht sichtbar oder nicht messbar waren.

Eine objektive Qualitätskontrolle ist wünschenswert. Nach den entsprechenden Vorgaben sollen in einem Thrombozytenkonzentrat beispielsweise mindestens 2·10¹¹ Thrombozyten und weniger als 1 Million Leukozyten bzw. 3 Millionen Resterythrozyten enthalten sein. Der pH-Wert eines Thrombozytenkonzentrates soll zwischen 6,4 und 7,8 liegen. Das Produkt muss bis zum Ende der maximalen Haltbarkeit steril sein.

Eine Einhaltung dieser Vorgaben kann quantitativ überprüft werden. Hierzu werden beispielsweise in Mikroskopieverfahren Zählungen der Thrombozyten, Erythrozyten und Leukozyten vorgenommen.

Herkömmliche Verfahren zur quantitativen Qualitätskontrolle von Blutprodukten, bei denen beispielsweise die Thrombozytenzahl, die Erythrozytenzahl, der Proteingehalt, der pH-Wert und/oder der Kaliumgehalt bestimmt wird, sind zeitaufwändig und teuer, insbesondere weil diese Proben nicht mehr für die Transfusion verwendet werden dürfen. Gemäß der Hämotherapie-Richtlinie der Bundesärztekammer (Stand 2010) müssen 1 % aller Blutproruckte, mindestens jedoch 4 Beutel für die Qualitätskontrolle einbehalten werden. Für die Überprüfung der Sterilität muss ein Anteil von 0,4·√n der Produkte überprüft werden und ist somit ebenfalls für eine Verwendung verloren. Darüber hinaus liefern herkömmliche Verfahren keine Aussagen oder nur sehr begrenzte Aussagen über die Funktionalität der in dem Blutprodukt enthaltenen Zellen. Der Funktionalitätszustand bzw. die Haltbarkeitsdauer der Blutprodukte könnten auch mit dem Befinden bzw. Zustand des Spenders in Zusammenhang stehen. Spenderspezifische Möglichkeiten zur Qualitätsüberprüfungen sind derzeit nicht bekannt.

Die Grundlagenstudie von Dasgupta et al. (J Biomed Opt. 2011. 16:077009) analysiert die Sauerstoffbeladungsfähigkeit von roten Blutzellen in Malaria-Patienten mittels Raman-Spektroskopie.

DE69734363 untersucht getrocknete Blutzellen mittels Fourier-Transformations-Infrarot-Spektroskopie.

### ZUSAMMENFASSUNG DER ERFINDUNG

Aufgabe der Erfindung ist es, Verfahren und Vorrichtungen anzugeben, die eine objektive Überprüfung von Blut erlauben. Ausführungsbeispielen liegt insbesondere die Aufgabe zugrunde, Verfahren und Vorrichtungen anzugeben, die eine objektive Überprüfung von Blutprodukten erlauben. Unter Blutprodukten werden dabei insbesondere Beutel mit einem Erythrozytenkonzentrat, Beutel mit Thrombozytenkonzentrat, Beutel mit Granulozytenkonzentrat, Beutel mit Leukozytenkonzentrat, Vollblutkonserven oder Blutplasmakonserven verstanden.

Es werden Verfahren und Vorrichtungen mit den in den unabhängigen Ansprüchen definierten Merkmalen angegeben. Die abhängigen Ansprüche definieren bevorzugte und vorteilhafte Ausführungsbeispiele.

Bei einem Verfahren zur Qualitätskontrolle eines Blutprodukts, das ein Erythrozytenkonzentrat, ein Thrombozytenkonzentrat, ein Granulozytenkonzentrat, ein Leukozytenkonzentrat, Vollblut und/oder Blutplasma umfasst, wird ein Raman-Spektrum durch Raman-Spektroskopie einer Probe des Blutprodukts erfasst. Anhand einer Auswertung des Raman-Spektrums wird bestimmt, ob das Blutprodukt für eine Transfusion verwendbar ist.

Bei der Erfassung des Raman-Spektrums an der Probe kann die Flüssigkeit selbst gemessen werden (beispielsweise der Kaliumgehalt und/oder Proteingehalt).

Erfindungsgemäß werden die zellulären Bestandteile wie Erythrozyten, Leukozyten, Granulozyten oder Thrombozyten erkannt und hinsichtlich ihrer Funktionalität untersucht. Diese in Lösung befindlichen Bestandteile werden erfindungsgemäß einzeln oder in einer Gruppe mit einigen Zellen in einer optischen Falle gefangen und durch Erfassung des Raman-Spektrums gemessen.

Das Raman-Spektrum kann auch an einer Probe erfasst werden, die ein Mikrotropfen ist, oder das Raman-Spektrum kann an einer Probe erfasst werden, die der eingetrocknete Überrest eines Tropfens nach Verdunsten ist (nicht Teil der Erfindung).

Die Probe kann auch ein Pellet sein, das durch Zentrifugation erzeugt wird und an dem das Raman-Spektrum erfasst wird (nicht Teil der Erfindung).

Die Verwendung der Raman-Spektroskopie erlaubt eine Überprüfung, ob die relevanten Zelltypen vorhanden sind und eine Bestimmung der biologischen Funktionalität. Insbesondere kann ermittelt werden, ob und ggf. welcher Anteil von Erythrozyten, Leukozyten, Granulozyten oder Thrombozyten einer molekularen Veränderung unterliegt. Beispielhaft für eine derartige molekulare Veränderung ist ein Zelltod (Apoptose oder Nekrose). Durch die Auswertung des Raman-Spektrums können auch Veränderungen der Zusammensetzung oder der Mengenverhältnisse und Konzentration der Biomoleküle in der Zelle erkannt werden, die eine Funktionalität der Zelle einschränken. Dadurch können Rückschlüsse auf die Verwendbarkeit und Funktionalität des Blutprodukts gezogen werden.

Die Verwendung der Raman-Spektroskopie erlaubt auch eine Bestimmung, ob in der Konserve Keime oder Bakterien vorhanden sind. Es können auch befallene Zellen, z.B. befallene Erythrozyten erkannt werden, beispielsweise bei Malaria. Darüber hinaus können aus dem Raman-Spektrum auch die Arten der Verunreinigungen der Konserve bestimmt werden und/oder die Anzahl der Verunreinigungen abgeschätzt werden.

Die Auswertung kann automatisch durch eine Auswerteeinrichtung erfolgen. Die Auswerteeinrichtung ist ein Computer.

Die Auswerteeinrichtung umfasst einen oder mehrere Prozessoren oder Controller.

Die Auswerteeinrichtung kann als ein Ergebnis der Auswertung eine optische und/oder akustische Ausgabe erzeugen, die anzeigt, ob das Blutprodukt noch für eine Transfusion verwendbar ist. Die Auswerteeinrichtung kann als ein Ergebnis der Auswertung Informationen über den Zustand zellulärer Bestandteile des Blutprodukts und/oder Informationen über in dem Blutprodukt enthaltene Verunreinigungen ausgeben.

Bei der Auswertung des Raman-Spektrums wird eine Spektralanalyse des Raman-Spektrums vorgenommen.

Durch die Auswertung des Raman-Spektrums kann ein Erythrozyten-Zerfall oder ein Thrombozyten-Zerfall, ein Erythrozyten-Zelltod oder ein Thrombozyten-Zelltod oder eine andere funktionelle Veränderung eines zellulären Bestandteils des Blutprodukts erkannt werden. Durch die Auswertung des Raman-Spektrums kann eine Einschränkung der Funktionalität von Erythrozten, Thrombozyten, Granulozyten oder Leukozyten erkannt werden.

Um zu erkennen, welcher Anteil von Zellen eines Zelltyps, z.B. welcher Anteil von Erythrozten, Thrombozyten, Granulozyten oder Leukozyten, einer funktionellen Veränderung unterliegt, die das Blutprodukt ungeeignet für eine Transfusion macht, wird automatisch eine Clusteranalyse mehrerer Raman-Spektren von Zellen dieses Zelltyps ausgeführt. Die Clusteranalyse kann eine Hauptkomponentenanalyse sein. Durch die Clusteranalyse kann quantitativ erkannt werden, ob Zellen einem Cluster intakter Zellen oder einem anderen Cluster funktionell beeinträchtigter Zellen zugeordnet werden müssen. Die Anzahl der Zellen in den unterschiedlichen Clustern gibt Information, welcher Anteil von Zellen des Zelltyps der funktionellen Veränderung unterliegt.

Für Blutprodukte, die mehrere unterschiedliche zelluläre Bestandteile des Bluts umfassen, kann durch eine Clusteranalyse nicht nur zwischen intakten Zellen und funktionell veränderten Zellen, sondern auch zwischen unterschiedlichen Zelltypen unterschieden werden.

Eine Zuordnung zu unterschiedlichen Zelltypen wird bei einer Clusteranalyse oder bei einer anderen Analyse der erfassten Raman-Spektren anhand unterschiedlicher Wellenzahlbereiche erfolgen. Zur Erkennung von Thrombozyten und/oder zur Erkennung funktioneller Veränderungen von Thrombozyten kann beispielsweise wenigstens eine Wellenzahl im Wellenzahlbereich von 1296 cm⁻¹ bis 1333 cm⁻¹ ausgewertet werden, um zu bestimmen, ob das Blutprodukt für eine Transfusion verwendbar ist.

Zur Erkennung von Erythrozyten und/oder zur Erkennung funktioneller Veränderungen von Erythrozyten kann beispielsweise wenigstens eine Wellenzahl aus einem oder mehreren der Wellenzahlbereiche von 1650 bis 1600 cm⁻¹, von 1350 bis 1250 cm⁻¹, von 1180 cm⁻¹ bis 1120 cm⁻¹, von 1100 cm⁻¹ bis 1050 cm⁻¹, von 930 cm⁻¹ bis 890 cm⁻¹ oder von 700 cm⁻¹ bis 650 cm⁻¹ ausgewertet werden, um zu bestimmen, ob das Blutprodukt für eine Transfusion verwendbar ist.

Zur Durchführung der Clusteranalyse müssen nicht notwendig die genannten Wellenzahlbereiche ausgewertet werden, sondern es können auch andere Hauptkomponenten ausgewertet werden.

Bei der Auswertung des Raman-Spektrums können ein oder mehrere Ramanpeaks identifiziert und optional weiter ausgewertet werden, die roten Blutkörperchen zugeordnet sind. Das Raman-Spektrum kann bei wenigstens einer und optional bei mehreren Wellenzahlen ausgewertet werden, die ausgewählt sind aus der Gruppe bestehend aus 669 cm⁻¹, 750 cm⁻¹, 752 cm⁻¹, 999 cm⁻¹, 1122 cm⁻¹, 1210 cm⁻¹, 1444 cm⁻¹, 1543 cm⁻¹ und 1617 cm⁻¹.

Bei der Auswertung des Raman-Spektrums kann beispielsweise ein Ramanpeak, der Guanin zugeordnet ist, erkannt werden. Es kann ein spektrales Gewicht oder eine Breite des Ramanpeaks, der Guanin zugeordnet ist, ermittelt werden.

Alternativ oder zusätzlich kann bei der Auswertung des Raman-Spektrums beispielsweise ein Ramanpeak, der Desoxyribonukleinsäure zugeordnet ist, erkannt werden. Es kann ein spektrales Gewicht oder eine Breite des Ramanpeaks, der Desoxyribonukleinsäure zugeordnet ist, ermittelt werden. Bei dem Ramanpeak kann es sich um ein Amid-III-Band einer α-Helix handeln.

Alternativ oder zusätzlich kann bei der Auswertung des Raman-Spektrums beispielsweise ein Ramanpeak, der einem Erythrozyten-Zerfall, einem Erythrozyten-Zelltod oder einer anderen funktionellen Beeinträchtigung von Erythrozyten zugeordnet ist, erkannt werden. Es kann ein spektrales Gewicht oder eine Breite des Ramanpeaks, der einem Erythrozyten-Zelltod zugeordnet ist, ermittelt werden.

Alternativ oder zusätzlich kann bei der Auswertung des Raman-Spektrums beispielsweise ein Ramanpeak, der einem Thrombozyten-Zerfall, einem Thrombozyten-Zelltod oder einer anderen funktionellen Beeinträchtigung von Thrombozyten zugeordnet ist, erkannt werden. Es kann ein spektrales Gewicht oder eine Breite des Ramanpeaks, der einem Thrombozyten-Zelltod zugeordnet ist, ermittelt werden.

Zum Erkennen eines oder mehrerer der genannten Ramanpeaks kann das Raman-Spektrum in einem vorgegebenen Wellenzahlintervall ausgewertet werden. Beispielsweise kann das Raman-Spektrum in einem Wellenzahlintervall von 1296 cm⁻¹ bis 1333 cm⁻¹ ausgewertet werden, um zu bestimmen, ob das Blutprodukt für eine Transfusion verwendbar ist.

Das Raman-Spektrum kann einer Spektralanalyse unterzogen werden. Beispielsweise kann eine Analyse von Mittelwertspektren, eine Hauptkomponentenanalyse und/oder eine Stützvektormaschine (SVM, "Support Vector Machine") verwendet werden, um zu bestimmen, ob das Blutprodukt für eine Transfusion verwendbar ist. Zum Erfassen des Raman-Spektrums wird wenigstens eine Zelle der Probe in einer optischen Falle gefangen, wobei die Probe Blutzellen enthält, die sich in Lösung befinden. Auf diese Weise können auch Zellen, die sich in Lösung befinden, spektroskopisch erfasst werden.

Falls die Probe die Form eines eingetrockneten Tropfens oder Pellets aufweist, aber auch um ein grobes Spektrum direkt in einer Flüssigkeitsmenge zu ermitteln, kann ein nicht fokussierter Strahl zur Messung eines Raman Spektrums verwendet werden. Dazu können beispielsweise lichtleiterbasierte Raman-Spektroskopiesysteme oder ein Raman-Spektroskopiesystem, das nicht optimal durch ein Objektiv fokussiert ist, verwendet werden, da solche Systeme meist flächig messen, wobei die abgedeckten Abmessungen bis hin zum Millimeterbereich reichen können (nicht Teil der Erfindung).

Zum Erfassen des Raman-Spektrums kann wenigstens ein optischer Lichtleiter, beispielsweise eine optische Faser, verwendet werden, um den Anregungsstrahl und/oder das Streulicht zu leiten.

Die optische Falle kann durch einen Anregungsstrahl der Raman-Spektroskopie erzeugt werden. Auf diese Weise kann mit einfachen Mitteln ein Raman-Signal mit gutem Signal-Rausch-Verhältnis erhalten werden.

Der Anregungsstrahl kann eine Wellenlänge zwischen 700 und 1064 nm aufweisen.

Die wenigstens eine Zelle kann ausgewählt sein aus einer Gruppe bestehend aus Erythrozyten, Thrombozyten und Leukozyten. Die wenigstens eine Zelle kann ein Granulozyt sein.

Durch die Raman-Spektroskopie kann eine Funktionalität von Erythrozyten und/oder Thrombozyten ausgewertet werden. Zusätzlich oder alternativ kann durch die Raman-Spektroskopie quantitativ erfasst werden, ob und ggf. in welcher Anzahl Verunreinigungen in dem Blutprodukt enthalten sind.

Zum Identifizieren von Bakterien und Keimen kann beispielsweise eine Blutproduktmenge aus der Konserve entnommen und weiter verarbeitet werden. Die Blutproduktmenge kann weiter verarbeitet werden, um etwaige vorhandene Verunreinigungen aufzukonzentrieren. Zum Aufkonzentrieren der Verunreinigungen können beispielsweise die zellulären Anteile wie Erythrozyten und/oder Thrombozyten und/oder Leukozyten wenigstens teilweise abgetrennt werden, bevor die Probe weiter konzentriert wird, beispielsweise durch Kombination mit Hydrogel. Die Probe kann dann durch Raman-Spektroskopie untersucht werden, um die Sterilität und somit die Verwendbarkeit des Blutprodukts zu überprüfen.

Die Probe kann aus dem Blutprodukt entnommen werden, um die Raman-Spektroskopie auszuführen. Das Blutprodukt kann einen Kunststoffbeutel aufweisen, in dem ein Erythrozytenkonzentrat, ein Thrombozytenkonzentrat, ein Granulozytenkonzentrat, ein Leukozytenkonzentrat oder Vollblut enthalten sein kann. Die Probe kann mit einer Spritze händisch oder durch einen Roboter automatisch aus dem Blutprodukt entnommen werden.

Die Raman-Spektroskopie kann durchgeführt werden, während das Blutprodukt in dem Beutel enthalten ist. Die Konserve steht nach der Qualitätskontrolle weiterhin als sterile Konserve zur Verfügung. Das Blutprodukt kann in einem Beutel enthalten sein, der für die Verwendung in einem Raman-Spektroskopiesystem ausgestaltet ist. Der Beutel kann aus einem Material bestehen, der für den Anregungsstrahl und das Raman-Streulicht der relevanten biologischen Objekte (beispielsweise Erythrozyten, Thrombozyten, Bakterien und/oder Keime) eine hohe Transmissivität aufweist. Der Beutel kann ein Fenster aus einem Material aufweisen, das für den Anregungsstrahl und das Raman-Streulicht der relevanten biologischen Objekte (beispielsweise Erythrozyten, Thrombozyten, Leukozyten, Bakterien und/oder Keime) eine hohe Transmissivität aufweist.

Eine Vorrichtung zur Qualitätskontrolle eines Blutprodukts, das ein Erythrozytenkonzentrat, ein Thrombozytenkonzentrat, ein Granulozytenkonzentrat, ein Leukozytenkonzentrat, Vollblut und/oder Blutplasma enthält, umfasst ein Raman-Spektroskopiesystem zum Erfassen eines Raman-Spektrums einer Probe des Blutprodukts. Die Vorrichtung umfasst eine Auswerteeinrichtung, die mit dem Ramanspektrometer gekoppelt ist und die eingerichtet ist, um anhand des erfassten Raman-Spektrums zu bestimmen, ob das Blutprodukt für eine Transfusion verwendbar ist.

Die Auswerteeinrichtung ist ein Computer. Die Auswerteeinrichtung umfasst einen oder mehrere Prozessoren oder Controller.

Die Auswerteeinrichtung kann als ein Ergebnis der Auswertung eine optische und/oder akustische Ausgabe erzeugen, die anzeigt, ob das Blutprodukt für eine Transfusion verwendbar ist.

Die Auswerteeinrichtung kann mit einem Bildsensor des Raman-Spektroskopiesystems gekoppelt sein. Der Bildsensor kann ein CCD-Sensor oder ein CMOS-Sensor sein.

Um zu erkennen, welcher Anteil von Zellen eines Zelltyps, z.B. welcher Anteil von Erythrozten, Thrombozyten, Granulozyten oder Leukozyten, einer funktionellen Veränderung unterliegt, die das Blutprodukt ungeeignet für eine Transfusion macht, ist die Auswerteeinrichtung eingerichtet, um Referenzspektren in einer Datenbank in einem Speicher des Computers zu hinterlegen, wobei die gespeicherten Referenzspektren eines oder mehrere der folgenden Elemente enthalten:
Lage von Raman-Peaks und/oder Wellenzahlbereichen von frischen Zellen eines oder mehrerer Zelltypen der genannten Blutprodukte;
Lage von Raman-Peaks und/oder Wellenzahlbereichen von funktionell veränderten Zellen eines oder mehrerer Zelltypen der genannten Blutprodukte;
   und
Informationen über Bereiche eines mehrdimensionalen Raumes einer Cluster-analyse, in denen die Referenzspektren jeweils angeordnet sind.

Die Auswerteeinrichtung kann weiterhin eingerichtet sein, um eine Clusteranalyse mehrerer Raman-Spektren von Zellen dieses Zelltyps auszuführen. Die Cluster-analyse kann eine Hauptkomponentenanalyse sein. Die Auswerteeinrichtung kann eingerichtet sein, um durch die Clusteranalyse quantitativ zu erkennen, ob Zellen einem Cluster intakter Zellen oder einem anderen Cluster funktionell beeinträchtigter Zellen zugeordnet werden müssen. Die Anzahl der Zellen in den unterschiedlichen Clustern kann von der Auswerteeinrichtung bestimmt werden, um zu ermitteln, welcher Anteil von Zellen des Zelltyps der funktionellen Veränderung unterliegt.

Für Blutprodukte, die mehrere unterschiedliche zelluläre Bestandteile des Bluts umfassen, kann die Auswerteeinrichtung durch eine Clusteranalyse nicht nur zwischen intakten Zellen und funktionell veränderten Zellen, sondern auch zwischen unterschiedlichen Zelltypen unterschieden werden.

Eine Zuordnung zu unterschiedlichen Zelltypen durch die Auswerteeinrichtung kann bei einer Clusteranalyse oder bei einer anderen Analyse der erfassten Raman-Spektren beispielsweise anhand unterschiedlicher Wellenzahlbereiche erfolgen. Zur Erkennung von Thrombozyten und/oder zur Erkennung funktioneller Veränderungen von Thrombozyten kann durch die Auswerteeinrichtung beispielsweise wenigstens eine Wellenzahl im Wellenzahlbereich von 1296 cm⁻¹ bis 1333 cm⁻¹ ausgewertet werden, um zu bestimmen, ob das Blutprodukt für eine Transfusion verwendbar ist.

Zur Erkennung von Erythrozyten und/oder zur Erkennung funktioneller Veränderungen von Erythrozyten durch die Auswerteeinrichtung kann beispielsweise wenigstens eine Wellenzahl aus einem oder mehreren der Wellenzahlbereiche von 1650 bis 1600 cm⁻¹, von 1350 bis 1250 cm⁻¹, von 1180 cm⁻¹ bis 1120 cm⁻¹, von 1100 cm⁻¹ bis 1050 cm⁻¹, von 930 cm⁻¹ bis 890 cm⁻¹ oder von 700 cm⁻¹ bis 650 cm⁻¹ ausgewertet werden, um zu bestimmen, ob das Blutprodukt für eine Transfusion verwendbar ist.

Zur Durchführung der Clusteranalyse durch die Auswerteeinrichtung müssen nicht notwendig die genannten Wellenzahlbereiche ausgewertet werden, sondern es können auch andere Hauptkomponenten ausgewertet werden.

Die Auswerteeinrichtung kann eingerichtet sein, um einen Ramanpeak, der einem Thrombozyten-Zelltod zugeordnet ist, zu erkennen, um zu bestimmen, ob die Konserve für eine Transfusion verwendbar ist. Die Auswerteeinrichtung kann eingerichtet sein, um einen Raman-Peak zu identifizieren, der eine Veränderung der Komposition der Biomoleküle in der Zelle repräsentiert, die die Funktionalität der Zelle einschränken.

Die Auswerteeinrichtung kann eingerichtet sein, um einen Raman-Peak, der roten Blutkörperchen zugeordnet ist, zu identifizieren und weiter auszuwerten.

Die Auswerteeinrichtung kann eingerichtet sein, um eine Spektralanalyse des Raman-Spektrums vorzunehmen.

Das Raman-Spektroskopiesystem kann einen Laser zum Erzeugen eines Anregungsstrahls für die Ramanspektroskopie umfassen. Der Laser kann Licht mit einer Wellenlänge zwischen 700 nm und 1064 nm erzeugen.

Das Raman-Spektroskopiesystem umfasst optische Komponenten, um durch den Anregungsstrahl eine optische Falle zu erzeugen, in der wenigstens eine Zelle der Probe zur Erfassung des Raman-Spektrums gefangen werden kann. Die optischen Komponenten können eine Linse umfassen. Die optischen Komponenten können einen Lichtleiter umfassen, in dem der Anregungsstrahl für die Raman-Spektroskopie geleitet wird. Durch das Fangen in einer optischen Falle können insbesondere auch Zellen, die beweglich sind, der Raman-Spektroskopie unterzogen werden.

Die Vorrichtung kann zur automatischen Durchführung des Verfahrens nach einem Ausführungsbeispiel eingerichtet sein.

Die Auswerteeinrichtung ist eingerichtet sein, um durch Auswertung eines oder mehrerer Raman-Spektren das Blutprodukt objektiv und quantitativ zu untersuchen. Es wird ein Vergleich mit in einer Datenbank hinterlegten Referenzspektren vorgenommen, um zu bestimmen, welche Zelltypen vorhanden sind und/oder um die Anzahl von Zellen eines oder mehreren Zelltypen zu quantifizieren und/oder um funktionelle Veränderungen von Zellen eines oder mehrerer Zelltypen zu erkennen. Alternativ oder zusätzlich kann eine Verarbeitung der Raman-Spektren, beispielsweise durch eine Clusteranalyse, vorgenommen werden, um unterschiedliche Zelltypen zu erkennen. Es kann ein Vergleich mit in einer Datenbank hinterlegten Referenzspektren vorgenommen werden, um Bakterien, Keime oder andere Verunreinigungen zu erkennen.

Die Auswerteeinrichtung kann eingerichtet sein, um erfasste Raman-Spektren des Blutprodukts nicht-flüchtig in einem Speicher zu speichern.

Die Auswerteeinrichtung kann einen Speicher umfassen, in dem Information über die Lage von Raman-Peaks unterschiedlicher Zelltypen eines Blutprodukts abgelegt sind. In dem Speicher kann Information über die Lage von Raman-Peaks von Erythrozyten gespeichert sein. Alternativ oder zusätzlich kann in dem Speicher Information über die Lage von Raman-Peaks von Thrombozyten gespeichert sein. Alternativ oder zusätzlich kann in dem Speicher Information über die Lage von Raman-Peaks von Granulozyten gespeichert sein. Alternativ oder zusätzlich kann in dem Speicher Information über die Lage von Raman-Peaks von Leukozyten gespeichert sein. Die Information über die Lage der Raman-Peaks kann in unterschiedlicher Weise gespeichert sein. Beispielsweise können die relevanten Wellenzahlbereiche für frische Zellen und für funktionell veränderte Zellen gespeichert sein. Es kann Information über Bereiche eines mehrdimensionalen Raums einer Cluster-Analyse, in denen die Spektren jeweils angeordnet sind, gespeichert sein.

Das Verfahren kann automatisch mit der Vorrichtung nach einem Ausführungsbeispiel ausgeführt werden.

Die Gewinnung der Blutprobe ist nicht Teil des beanspruchten Verfahrens.

### KURZE BESCHREIBUNG DER FIGUREN

FIG. 1 zeigt eine schematische Darstellung einer Vorrichtung 2.
FIG. 2 ist ein Flussdiagramm eines Verfahrens.
FIG. 3 zeigt eine schematische Darstellung eines Raman-Spektrums, das von Vorrichtungen und Verfahren ausgewertet wird
FIG. 4 zeigt eine schematische Darstellung eines Raman-Spektrums, das von Vorrichtungen und Verfahren ausgewertet wird
FIG. 5 zeigt eine schematische Darstellung einer statistischen Auswertung des Raman-Spektrums einer Probe.
FIG. 6 zeigt eine schematische Darstellung einer statistischen Auswertung des Raman-Spektrums einer weiteren Probe.
FIG. 7 zeigt eine schematische Darstellung einer statistischen Auswertung des Raman-Spektrums einer Probe.
FIG. 8 zeigt eine schematische Darstellung einer statistischen Auswertung des Raman-Spektrums einer weiteren Probe.
FIG. 9 zeigt eine schematische Darstellung von Raman-Spektren, die von Vorrichtungen und Verfahren ausgewertet werden
FIG. 10 zeigt eine schematische Darstellung der Raman-Spektroskopie in Kombination mit einer optischen Falle.
FIG. 11 zeigt eine schematische Darstellung einer Konserve, die für eine Durchführung des Verfahrens an der geschlossenen Konserve eingerichtet ist.

### BESCHREIBUNG VON AUSFÜHRUNGSBEISPIELEN

Die Merkmale der verschiedenen beschriebenen Ausführungsformen können miteinander kombiniert werden, sofern dies in der nachfolgenden Beschreibung nicht ausdrücklich ausgeschlossen ist.

Vorrichtungen und Verfahren nach Ausführungsbeispielen werden zur Qualitätskontrolle von Blutprodukten eingesetzt.

Der Begriff "Blutprodukt" beinhaltet dabei Konserven mit Erythrozytenkonzentrat, Konserven mit Thrombozytenkonzentrat, Konserven mit Granulozytenkonzentrat, Konserven mit Leukozytenkonzentrat, Konserven mit Vollblut und Konserven mit Blutplasma. Granulozytenkonzentrate enthalten neben Granulozyten auch andere Leukozyten, welche eine GvHD ("Graft versus Host")-Reaktion beim Empfänger hervorrufen können. Durch eine Bestrahlung wird die Vermehrungsfähigkeit der Lymphozyten irreversibel verhindert. Ein Leukozytenkonzentrat wird mittels Leukopherese aus Spenderblut gewonnen und enthält vor allem Granulozyten. Das Leukozytenkonzentrat ist nur wenige Stunden haltbar und wird zur Infektionsprophylaxe bei ausgeprägtem, aber reversiblem Leukozytenmangel verabreicht.

Bei Vorrichtungen und Verfahren nach Ausführungsbeispielen wird ein Raman-Spektrum einer Probe des Blutprodukts erfasst. Das Raman-Spektrum wird ausgewertet, um zu bestimmen, ob das Blutprodukt noch für eine Transfusion geeignet ist. Das Blutprodukt kann eine Blutkonserve sein, wobei beispielsweise ein Erythrozytenkonzentrat, ein Thrombozytenkonzentrat, ein Granulozytenkonzentrat, ein Leukozytenkonzentrat, Vollblut oder Blutplasma in einem geeigneten Behältnis enthalten ist.

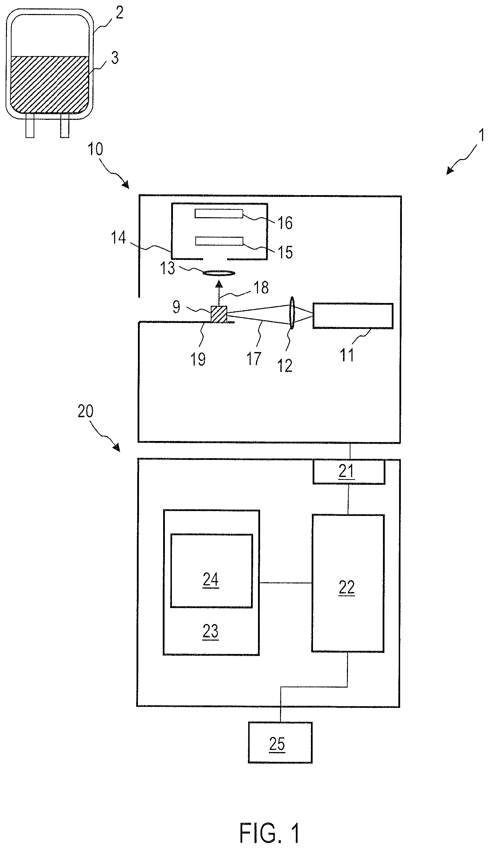

FIG. 1 ist eine schematische Darstellung einer Vorrichtung 1 nach einem Ausführungsbeispiel. Die Vorrichtung 1 ist eingerichtet, um zu bestimmen, ob eine Blutkonserve 2 für eine Transfusion geeignet ist. Die entsprechende Bestimmung erfolgt anhand eines Raman-Spektrums, das die Vorrichtung 1 erfasst und automatisch auswerten kann. Eine Probe eines Blutprodukt 3, bei dem es sich beispielsweise um ein Erythrozytenkonzentrat, ein Thrombozytenkonzentrat, ein Granulozytenkonzentrat, ein Leukozytenkonzentrat, Vollblut oder Blutplasma handeln kann, wird für eine Raman-Spektroskopie verwendet, um zu bestimmen, ob das Blutprodukt 3 für eine Transfusion verwendet werden darf. Die Herstellung der Blutkonserve 2, die nach herkömmlichen Techniken erfolgen kann, ist nicht Gegenstand der hier offenbarten Verfahren und Vorrichtungen.

Die Vorrichtung 1 umfasst ein Raman-Spektroskopiesystem 10 und eine Auswerteeinrichtung 20. Das Raman-Spektroskopiesystem 10 ist eingerichtet, um ein Raman-Spektrum einer Probe 9 des Blutprodukts 3 zu erfassen. Eine Menge des Blutprodukts 3 kann aus der Blutkonserve 2 entnommen werden und nach weiterer Verarbeitung als Probe 9 für das Raman-Spektroskopiesystem 10 bereitgestellt werden. Die Probe 9 enthält Blutzellen, die in einer Lösung beweglich sind. Die Probe 9 kann eingetrocknetes Blut oder ein Pellet enthalten, das aus dem Blutprodukt 3 erzeugt wird (nicht Teil der Erfindung).

Bei weiteren Ausgestaltungen kann das Raman-Spektroskopiesystem 10 auch so ausgestaltet sein, dass das Raman-Spektrum unmittelbar an der Blutkonserve 2 aufgenommen wird, ohne dass dieser zuvor eine Menge des Blutprodukts 3 entnommen werden muss. Es kann dann eine gesamte Blutkonserve in das Raman-Spektroskopiesystem eingeführt werden.

Das Raman-Spektroskopiesystem 10 umfasst einen Lichtquelle 11, die insbesondere ein Laser sein kann. Die Lichtquelle 11 ist eingerichtet, um einen Anregungsstrahl 17 auszugeben. Der Anregungsstrahl 17 kann beispielsweise eine Wellenlänge im Intervall zwischen 700 nm und 1064 nm aufweisen, z.B. ungefähr 785 nm. Ein Ramanspektrometer 14 empfängt an der Probe 9 durch Stokes-Prozesse und/oder Anti-Stokes-Prozesse gestreutes Licht 18. Das Ramanspektrometer 14 kann ein diffraktives Element 15 und einen Bildsensor 16 umfassen, um das Raman-Spektrum der Probe 9 zu erfassen. Das Raman-Spektroskopiesystem 10 kann in an sich bekannter Weise weitere Elemente umfassen, beispielsweise fokussierende optische Elemente 12, 13, die als Linsen ausgestaltet sein können, und/oder Blenden.

Die Vorrichtung 1 umfasst eine Auswerteeinrichtung 20. Die Auswerteeinrichtung 20 ist ein Computer sein oder umfasst einen Computer. Die Auswerteeinrichtung 20 ist mit dem Raman-Spektroskopiesystem 10 gekoppelt. Die Auswerteeinrichtung 20 kann die Erfassung des Raman-Spektrums durch das Raman-Spektroskopiesystem 10 steuern.

Die Auswerteeinrichtung 20 weist eine Schnittstelle 21 auf, um Daten von dem Bildsensor 16 des Raman-Spektroskopiesystems 10 zu empfangen. Die Auswerteeinrichtung weist eine integrierte Halbleiterschaltung 22 auf, die einen Prozessor oder Controller umfassen kann und die eingerichtet ist, um das erfasste Raman-Spektrum auszuwerten, um zu bestimmen. Die integrierte Halbleiterschaltung 22 ist eingerichtet, um anhand des Raman-Spektrums zu bestimmen, ob die Blutkonserve 2 noch für eine Transfusion verwendbar ist. Die integrierte Halbleiterschaltung 22 kann insbesondere eingerichtet sein, um durch Auswertung des Raman-Spektrums zu ermitteln, ob und in welchem Umfang die Funktionalität der Zellen beeinträchtigt ist. Die integrierte Halbleiterschaltung 22 kann eingerichtet sein, um durch Auswertung des Raman-Spektrums zu ermitteln, ob ein Zelltod von Erythrozyten und/oder Thrombozyten und/oder Granulozyten und/oder Leukozyten eingetreten ist. Die integrierte Halbleiterschaltung 22 kann eingerichtet sein, um durch Auswertung des Raman-Spektrums zu ermitteln, ob biologische Moleküle der Zellen vorhanden sind, die die Funktion der Zelle beeinträchtigen.

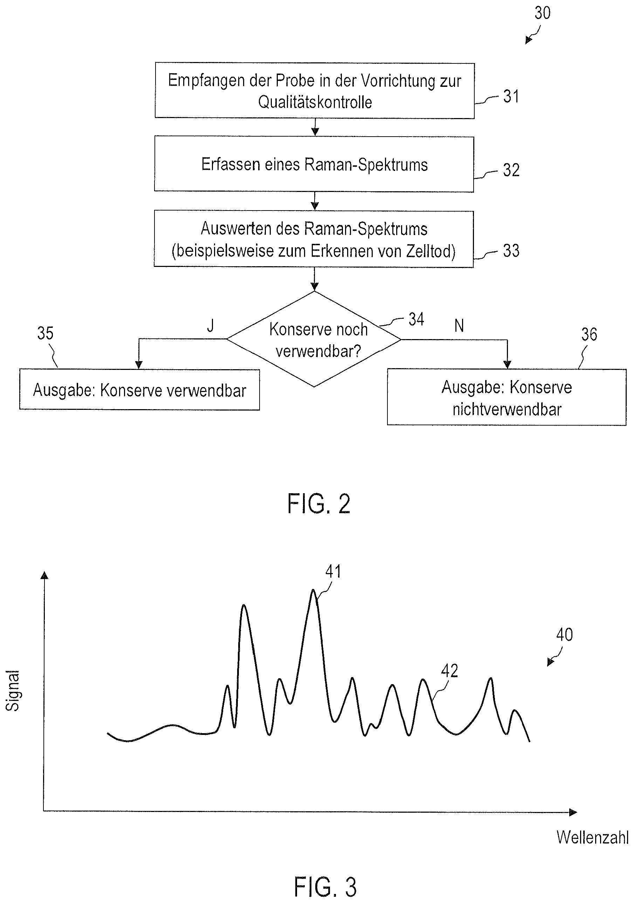

Wie unter Bezugnahme auf FIG. 2 bis FIG. 11 ausführlicher beschrieben wird, kann die integrierte Halbleiterschaltung 22 eingerichtet sein, um die Anwesenheit oder Abwesenheit bestimmter Ramanpeaks zu erkennen oder das spektrale Gewicht von Ramanpeaks zu bestimmen, die mit der Güte der Blutkonserve 2 zusammenhängen. Beispielsweise kann die integrierte Halbleiterschaltung 22 Ramanpeaks erkennen und/oder weiter auswerten, die roten Blutkörperchen zugeordnet sind oder die einem Zelltod von Erythrozyten und/oder Thrombozyten, Granulozyten, Leukozyten zugeordnet ist. Die integrierte Halbleiterschaltung 22 kann eingerichtet sein, um dazu beispielsweise das Raman-Spektrum in wenigstens einem vorgegebenen Wellenzahlintervall, z.B. das Raman-Spektrum im Wellenzahlbereich zwischen 1296 cm⁻¹ und 1333 cm⁻¹, auszuwerten, um zu bestimmen, ob das Blutprodukt 3 für eine Transfusion verwendbar ist.

Die integrierte Halbleiterschaltung 22 kann eingerichtet sein, um automatisch die Sterilität der Blutkonserve 2 durch eine Analyse des Raman-Spektrums zu überprüfen. Die integrierte Halbleiterschaltung 22 kann eingerichtet sein, um ein oder mehrere Ramanpeaks zu identifizieren, die Verunreinigungen, beispielsweise Bakterien oder Viren zugeordnet sind, um zu bestimmen, ob die Blutkonserve 2 steril ist.

Die Auswerteeinrichtung 20 kann einen Speicher 23 umfassen, in dem Vergleichsdaten 24 hinterlegt sind, die die integrierte Halbleiterschaltung 22 bei der Auswertung des Raman-Spektrums mit verwenden kann.

Information über die Lage und/oder das spektrale Gewicht unterschiedlicher Raman-Peaks für die unterschiedlichen Zelltypen eines oder mehrerer Blutprodukte können nichtflüchtig in dem Speicher 23 der Vorrichtung 1 gespeichert sein. Alternativ oder zusätzlich kann die Information über die Lage und/oder das spektrale Gewicht unterschiedlicher Raman-Peaks für die unterschiedlichen des Blutprodukts von der Vorrichtung 1 durch Methoden des beaufsichtigten Lernens ("Supervised Learning") oder anderer Techniken des maschinellen Lernens ermittelt werden.

Die Auswerteeinrichtung 20 kann eine optische und/oder akustische Ausgabeeinheit 25 umfassen, über die abhängig von der Analyse des Raman-Spektrums Information ausgegeben wird, die anzeigt, ob die Blutkonserve 2 noch verwendbar ist oder nicht. Die Ausgabeeinheit 25 kann auch baulich in ein Gehäuse der Auswerteeinrichtung 20 oder des Raman-Spektroskopiesystems 10 integriert sein.

Auch wenn die Auswerteeinrichtung 20 und das Raman-Spektroskopiesystem 10 in FIG. 1 schematisch als separate Einheiten dargestellt sind, können die Funktionen der Auswerteeinrichtung 20 auch in einem Gehäuse des Raman-Spektroskopiesystems 10 integriert sein. Das Raman-Spektroskopiesystem 10 und die Auswerteeinrichtung 20 können als mobile, insbesondere als tragbare Einheiten ausgestaltet sein.

FIG. 2 ist ein Flussdiagramm eines Verfahrens 30. Das Verfahren 30 kann von der Vorrichtung 1 vollautomatisch ohne dazwischen geschaltete Bedienhandlungen eines Benutzers ausgeführt werden oder kann abhängig von Benutzereingaben ausgeführt werden. Eine Probe 9 kann aus dem Blutprodukt 9 gewonnen werden. Beispielsweise kann unter Verwendung einer Spritze eine Menge des Blutprodukts 3 aus der Blutkonserve 2 entnommen werden. Die entnommene Menge des Blutprodukts 3 kann verdünnt werden, um die Probe 9 zu erzeugen. Es kann auch mehr als eine Probe erzeugt und mit Raman-Spektroskopie analysiert werden. Beispielsweise können von aus der Blutkonserve 2 entnommenen Blutproduktproben zunächst Verunreinigungen aufkonzentriert werden, beispielsweise durch Abtrennen von Erythrozyten und/oder Thrombozyten und Erzeugen von Bedingungen, die zu einer Vermehrung etwaiger vorhandener Verunreinigungen in der entnommenen Probe führen. Auf diese Weise kann die quantitative Bestimmung von Verunreinigungen zur Überprüfung der Sterilität verbessert werden. Die Probe 9 kann auch in fester Form vorliegen (nicht Teil der Erfindung).

Beispielsweise kann die entnommene Menge des Blutprodukts 3 ein Tropfen sein, den man zunächst verdunsten lässt, um das nach Verdunstung verbleibende Material als Probe 9 zu nutzen. Die Probe 9 kann ein Pellet sein.

Bei Schritt 31 empfängt die Vorrichtung 1 die Probe 9. Beispielsweise kann ein Probenhalter 19 automatisch eingefahren werden, nachdem die Probe 9 dort platziert wurde.

Bei Schritt 32 wird ein Raman-Spektrum der Probe 9 erfasst. Die Lichtquelle 11 wird so gesteuert, dass ein Anregungsstrahl 17 erzeugt wird. Der Anregungsstrahl 17 oder ein von dem Anregungsstrahl 17 verschiedener Strahl elektromagnetischer Strahlung erzeugt eine optische Falle,

in der Zellen der Probe 9 zur Raman-Spektroskopie gefangen werden, da die Probe 9 flüssig ist.

Es können auch mehrere Raman-Spektren erfasst werden. Beispielsweise können mehrere Raman-Spektren an derselben Probe oder an unterschiedlichen Proben erfasst werden, um aus einem Raman-Spektrum zu ermitteln, ob die Erythrozyten und/oder Thrombozyten und/oder Leukozyten leben, und um aus einem anderen Raman-Spektrum zu ermitteln, wie viele Verunreinigungen die Blutkonserve enthält.

Bei Schritt 33 wertet die Auswerteeinrichtung 20 das erfasste Raman-Spektrum aus. Die Auswerteeinrichtung 20 kann Ramanpeaks identifizieren, die beispielsweise Guanin, Desoxyribonukleinsäure oder einem Zelltod von Erythrozyten oder Thrombozyten zugeordnet sind. Die Auswerteeinrichtung 20 kann Ramanpeaks identifizieren, die roten Blutkörperchen zugeordnet sind. Die Auswerteeinrichtung 20 kann eine statistische Auswertung des Raman-Spektrums vornehmen, beispielsweise durch eine Spektralanalyse.

Bei Schritt 34 wird abhängig von der Auswertung des Raman-Spektrums überprüft, ob die Blutkonserve 2 mit dem Blutprodukt 3 für eine Transfusion verwendet werden darf. Dazu kann die Auswerteeinrichtung 20 beispielsweise ein spektrales Gewicht eines Ramanpeaks, der dem Zelltod von Erythrozyten oder Thrombozyten zugeordnet ist, mit einem Schwellenwert vergleichen. Abhängig davon, ob das spektrale Gewicht von Ramanpeaks, das mit dem Zelltod von Erythrozyten oder Thrombozyten assoziiert ist, größer als der Schwellenwert ist, kann bestimmt werden, dass die Probe nicht mehr für die Transfusion geeignet ist. Alternativ oder zusätzlich können Datenpunkte, die durch eine Spektralanalyse des Raman-Spektrums ermittelt wurden, verwendet werden, um zu bestimmen, ob die Blutkonserve bereits so alt ist oder so gelagert wurde, dass sie nicht mehr für eine Transfusion verwendbar ist. Alternativ oder zusätzlich kann durch die Analyse des Raman-Spektrums bestimmt werden, ob die Probe noch steril ist. Es kann eine Art und eine Anzahl von Verunreinigungen abgeschätzt werden.

Bei Schritt 35 oder Schritt 36 wird eine Ausgabeeinheit so angesteuert, dass Information angibt, die anzeigt, ob die Blutkonserve noch verwendbar ist oder nicht.

FIG. 3 zeigt schematisch ein Raman-Spektrum 40, das von der Auswerteeinrichtung 20 ausgewertet wird. Das Raman-Spektrum 40 kann mehrere Ramanpeaks 41, 42 aufweisen, die von der Auswerteeinrichtung 20 automatisch identifiziert werden können und die Information darüber geben, ob die Blutkonserve noch verwendbar ist. Die Ramanpeaks 41, 42 können beispielsweise mit Stoffen assoziiert sein, die einen Zelltod (z.B. eine Apoptose) von Erythrozyten oder Thrombozyten anzeigen. Die Ramanpeaks 41, 42 können roten Blutkörperchen zugeordnet sein. Beispielsweise können einer oder mehrere der analysierten Ramanpeaks bei Wellenzahlen liegen, die ausgewählt sind aus der Gruppe bestehend aus 669 cm⁻¹, 750 cm⁻¹, 752 cm⁻¹, 999 cm⁻¹, 1122 cm⁻¹, 1210 cm⁻¹, 1444 cm⁻¹, 1543 cm⁻¹ und 1617 cm⁻¹.

Die Auswerteeinrichtung 20 kann gezielt nur ein vorgegebenes Wellenzahlintervall oder mehrere vorgegebene Wellenzahlintervalle des Raman-Spektrums 40 analysieren, um zu bestimmen, ob die Blutkonserve noch für eine Transfusion geeignet ist. Die Auswerteeinrichtung 20 kann beispielsweise ein Wellenzahlintervall von 1200 cm⁻¹ bis 1400 cm⁻¹ analysieren. Die Auswerteeinrichtung 20 kann beispielsweise ein Wellenzahlintervall von 1276 cm⁻¹ bis 1333 cm⁻¹ analysieren. Andere Wellenzahlbereiche können verwendet werden, beispielsweise Wellenzahlbereiche, in denen charakteristische Ramanpeaks roter Blutkörperchen liegen.

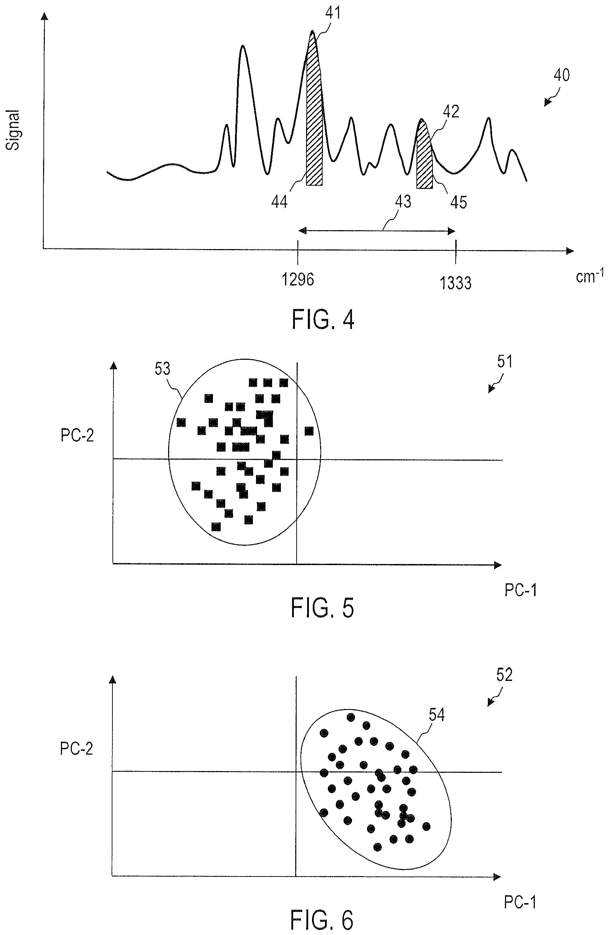

FIG. 4 zeigt schematisch das Raman-Spektrum 40, das von der Auswerteeinrichtung 20 ausgewertet wird. Nur beispielhaft dargestellt ist ein Wellenzahlbereich von 1276 cm⁻¹ bis 1333 cm⁻¹, in dem ein oder mehrere Ramanpeaks 41, 42 liegen, die von der Auswerteeinrichtung 20 identifiziert werden. Eine weitere Auswertung durch eine Spektralanalyse kann erfolgen. Beispielsweise kann die Auswerteeinrichtung 20 ein Integral 44, 45 des Signals 40 für die jeweiligen Ramanpeaks 41, 42 bestimmen, z.B. durch numerische Integration oder Aufsummieren der Signalbeiträge in mehreren diskreten Wellenzahlbändern, um ein Maß für das spektrale Gewicht der Ramanpeaks 41, 42 zu erhalten. Weitere Auswertungen können vorgenommen werden, beispielsweise durch eine Hauptkomponentenanalyse des Raman-Spektrums 40, durch Auswertung des Mittelwertspektrums oder durch eine SVM, ohne darauf beschränkt zu sein.

FIG. 5 und FIG. 6 veranschaulichen beispielhafte Ergebnisse einer Hauptkomponenteanalyse oder einer anderen Clusteranalyse, die von der Auswerteeinrichtung 20 durchgeführt wird, um zu bestimmen, ob die Blutkonserve noch für eine Transfusion geeignet ist. Dabei wird die Hauptkomponentenanalyse für ein Raman-Spektrum oder mehrere Raman-Spektren ausgeführt, die von der Probe 9 erfasst wurden. Die Datenpunkte sind gemäß einem Paar der unterschiedliche Hauptkomponenten PC-1 und PC-2 dargestellt. FIG. 5 zeigt die Datenpunkte 51, die durch Verarbeitung des Raman-Spektrums einer relativ frischen Blutkonserve gewonnen wurden. FIG. 6 zeigt Datenpunkte 52, die durch Verarbeitung des Raman-Spektrums einer Blutkonserve gewonnen wurden, die länger und/oder nicht bei der richtigen Temperatur gelagert wurde.

Wie aus einem Vergleich von FIG. 5 und FIG. 6 erkennbar, verschieben sich die durch die Hauptkomponentenanalyse gewonnenen Datenpunkte abhängig davon, ob die Blutkonserve noch für die Transfusion geeignet ist oder nicht. Entsprechend kann die Auswerteeinrichtung 20 anhand der Hauptkomponentenanalyse eines Raman-Spektrums automatisch bestimmen, ob die jeweils getestete Blutkonserve 2 noch für eine Transfusion geeignet ist.

Es können unterschiedliche Zonen 53, 54 in einem N-dimensionalen Raum definiert werden, in dem die Punkte der Clusteranalyse abhängig davon angeordnet sind, ob zelluläre Bestandteile des Blutprodukts intakte Zellen oder funktionell beeinträchtigte Zellen sind.

Die Datenpunkte, die frischen und somit intakten zellulären Bestandteilen zugeordnet sind, können in einem Bereich 53 angeordnet sein. Die Datenpunkte, die alten und funktionell veränderten zellulären Bestandteilen zugeordnet sind, können in einem davon verschiedenen Bereich 54 des N-dimensionalen Raums angeordnet sein. Die Dimension N des Raums, in dem die Clusteranalyse ausgeführt wird, kann größer als zwei, insbesondere viel größer als zwei sein.

Um zu erkennen, welcher Anteil von Zellen eines Zelltyps, z.B. welcher Anteil von Erythrozten, Thrombozyten, Granulozyten oder Leukozyten, einer funktionellen Veränderung unterliegt, die das Blutprodukt ungeeignet für eine Transfusion macht, kann die Auswerteeinrichtung 20 somit eingerichtet sein, um eine Clusteranalyse mehrerer Raman-Spektren von Zellen dieses Zelltyps auszuführen. Die Clusteranalyse kann eine Hauptkomponentenanalyse sein. Die Auswerteeinrichtung 20 kann eingerichtet sein, um durch die Clusteranalyse quantitativ zu erkennen, ob Zellen einem Cluster 51 intakter Zellen oder einem anderen Cluster 52 funktionell beeinträchtigter Zellen zugeordnet werden müssen. Die Anzahl der Zellen in den unterschiedlichen Clustern 51, 52 kann von der Auswerteeinrichtung 20 bestimmt werden, um zu ermitteln, welcher Anteil von Zellen des Zelltyps der funktionellen Veränderung unterliegt.

Für Blutprodukte, die mehrere unterschiedliche zelluläre Bestandteile des Bluts umfassen, kann die Auswerteeinrichtung 20 durch eine Clusteranalyse nicht nur zwischen intakten Zellen und funktionell veränderten Zellen, sondern auch zwischen unterschiedlichen Zelltypen unterschieden werden. Für jeden von mehreren unterschiedlichen Zelltypen kann dann ermittelt werden, welcher Anteil von Zellen dieses Zelltyps einer funktionellen Veränderung unterliegt.

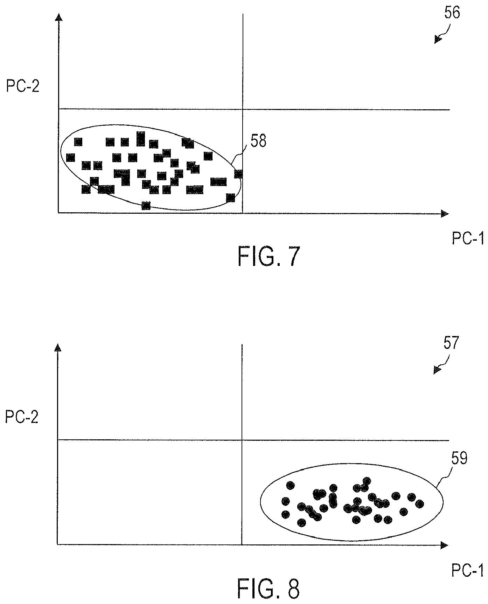

FIG. 7 und FIG. 8 zeigen die Ergebnisse einer Clusteranalyse, z.B. einer Hauptkomponentenanalyse, für einen weiteren Zelltyp, der von dem in FIG. 5 und FIG. 6 untersuchten weiteren verschieden ist. FIG. 7 und FIG. 8 zeigen beispielhaft Ergebnisse für Erythrozyten zum Vergleich mit beispielhaften Ergebnissen für Thrombozyten, wie sie in FIG. 5 und FIG. 6 dargestellt sind. FIG. 7 und FIG. 8 zeigen Anordnungen von Datenpunkten, von denen jeder einem Raman-Spektrum zugeordnet ist, in einem N-dimensionalen Datenraum bei einer Hauptkomponentenanalyse. Andere Techniken der Clusteranalyse können eingesetzt werden.

FIG. 7 zeigt beispielhaft Datenpunkte für Raman-Spektren von frischen, intakten Zellen. FIG. 8 zeigt beispielhaft Datenpunkte für Raman-Spektren von alten, funktionell veränderten Zellen. Wie unter Bezugnahme auf FIG. 5 und FIG. 6 erläutert, können durch die Clusteranalyse Daten 56, die intakten Zellen zugeordnet werden könne, und Daten 57, die funktionell veränderten Zellen zugeordnet werden können, unterschieden werden.

Datenpunkte, die zu Zellen unterschiedlicher Zelltypen gehören, liegen bei der Clusteranalyse auch in unterschiedlichen Bereichen des Datenraums. Somit kann eine Unterscheidung unterschiedlicher Zellen erfolgen. Eine Zuordnung von Datenpunkten zu zellulären Bestandteilen wie Erythrozten, Thrombozyten, Granulozyten oder Leukozyten kann durch einen Abgleich mit der Lage von Datenpunkten, die intakten Zellen zugeordnet sind, erfolgen. Derartige Referenzdaten können nichtflüchtig in der Vorrichtung 1 gespeichert sein.

Wie beispielhaft in FIG. 7 im Vergleich zu FIG. 5 dargestellt ist, unterscheidet sich der Bereich 58, in dem Datenpunkte für Zellen eines zweiten Zelltyps bei der Clusteranalyse liegen, von dem Bereich 53, in dem Datenpunkte für Zellen eines zweiten Zelltyps bei der Clusteranalyse liegen.

Funktionelle Veränderungen der Zellen, z.B. aufgrund von Alter oder Aufbewahrungsbedingungen des Blutprodukts, führen zu einer Verschiebung der Datenpunkte bei der Clusteranalyse von dem Bereich 58 in einen davon verschiedenen Bereich 59.

Die Auswerteeinrichtung 20 kann bei einer Clusteranalyse oder bei einer anderen Analyse der erfassten Raman-Spektren eine Zuordnung zu unterschiedlichen Zelltypen beispielsweise anhand unterschiedlicher Wellenzahlbereiche erfolgen, in denen die Raman-Spektren für Zellen unterschiedlicher Zelltypen jeweils ein charakteristisches Verhalten aufweisen. Zur Erkennung von Thrombozyten und/oder zur Erkennung funktioneller Veränderungen von Thrombozyten kann durch die Auswerteeinrichtung 20 beispielsweise wenigstens eine Wellenzahl im Wellenzahlbereich von 1296 cm⁻¹ bis 1333 cm⁻¹ ausgewertet werden, um zu bestimmen, ob das Blutprodukt für eine Transfusion verwendbar ist.

Zur Erkennung von Erythrozyten und/oder zur Erkennung funktioneller Veränderungen von Erythrozyten durch die Auswerteeinrichtung 20 kann beispielsweise wenigstens eine Wellenzahl aus einem oder mehreren der Wellenzahlbereiche von 1650 bis 1600 cm⁻¹, von 1350 bis 1250 cm⁻¹, von 1180 cm⁻¹ bis 1120 cm⁻¹, von 1100 cm⁻¹ bis 1050 cm⁻¹, von 930 cm⁻¹ bis 890 cm⁻¹ oder von 700 cm⁻¹ bis 650 cm⁻¹ ausgewertet werden, um zu bestimmen, ob das Blutprodukt für eine Transfusion verwendbar ist.

Abhängig von dem Anteil von Zellen eines oder mehrerer Zelltypen, die einer funktionellen Veränderung unterliegen, kann die Auswerteeinrichtung 20 automatisch bestimmen, ob das Blutprodukt für eine Transfusion geeignet ist.

Es kann ein Schwellenwert für einen Zelltyp des Blutprodukts nichtflüchtig in der Vorrichtung 1 gespeichert sein. Übersteigt der Anteil von Zellen des Zelltyps, die nach dem Ergebnis der Hauptkomponentenanalyse oder einer anderen Clusteranalyse funktionell beeinträchtigt sind, den Schwellenwert, erkennt die Auswerteeinrichtung 20 automatisch, dass das Blutprodukt nicht für die Transfusion geeignet ist.

Es kann ein weiterer Schwellenwert für einen weiteren Zelltyp des Blutprodukts nicht-flüchtig in der Vorrichtung 1 gespeichert sein. Übersteigt der Anteil von Zellen des weiteren Zelltyps, die nach dem Ergebnis der Hauptkomponentenanalyse oder einer anderen Clusteranalyse funktionell beeinträchtigt sind, den weiteren Schwellenwert, erkennt die Auswerteeinrichtung 20 automatisch, dass das Blutprodukt nicht für die Transfusion geeignet ist.

Der Zelltyp und der weitere Zelltyp können beide ausgewählt sein aus einer Gruppe bestehend aus Erythrozyten, Thrombozyten, Granulozyten und Leukozyten.

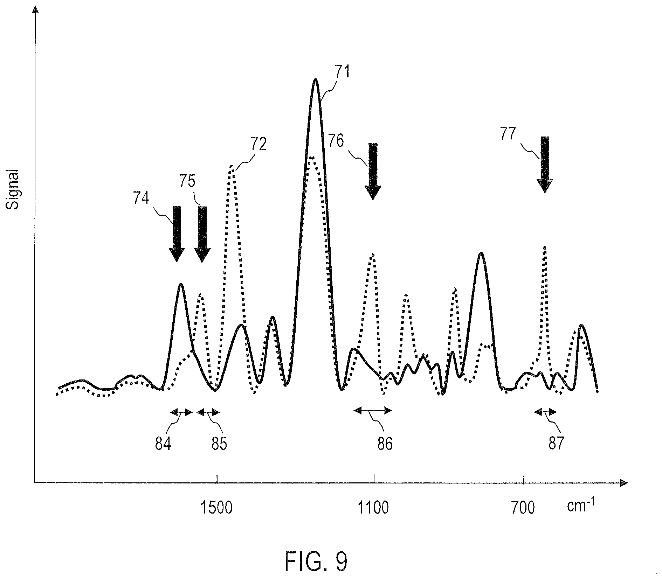

FIG. 9 zeigt ein Raman-Spektrum 71 von Thrombozyten und ein Raman-Spektrum 72 von Erythrozyten. Jeweils dargestellt sind Mittelwert-Spektren nach einer Lagerung des Blutprodukts.

Die Lage von Raman-Peaks erlaubt eine Unterscheidung unterschiedlicher Zelltypen. Beispielsweise weist das Raman-Spektrum 71 von Thrombozyten Raman-Peaks bei einer Wellenzahl oder mehreren Wellenzahlen 74 auf, die eine Unterscheidung von Thrombozyten und anderen Zelltypen erlauben. In einem Wellenzahlintervall 84 oder in mehreren Wellenzahlintervallen, das beispielsweise von 1550 cm⁻¹ bis 1590 cm⁻¹ reichen kann, kann aus der Anwesenheit eines Raman-Peaks eine Zelle vom Zelltyp Thrombozyten erkannt werden.

Das Raman-Spektrum 72 von Erythrozyten weist Raman-Peaks bei Wellenzahlen 75, 76, 77 auf, die die eine Unterscheidung von Thrombozyten und anderen Zelltypen erlauben. In einem Wellenzahlintervall oder in mehreren Wellenzahlintervallen 85, 86, 87 kann aus der Anwesenheit eines Raman-Peaks eine Zelle vom Zelltyp Erythrozyten erkannt werden. Die ein oder mehreren Wellenzahlintervalle 85, 86, 87 können ausgewählt sein aus der Gruppe bestehend aus einem Wellenzahlintervall von 1480 cm⁻¹ bis 1550 cm⁻¹, einem Wellenzahlintervall von 1050 cm⁻¹ bis 1120 cm⁻¹ und einem Wellenzahlintervall von 600 cm⁻¹ bis 700 cm⁻¹.

Zur Unterscheidung unterschiedlicher Zelltypen und der funktionellen Veränderungen, denen die Zellen jeweils unterliegen, kann eine Cluster-Analyse ausgeführt werden, wie dies oben bereits beschrieben wurde.

Zusätzlich zu einer Bestimmung der Anteile zellulärerer Bestandteile der Blutprodukte, die funktionellen Veränderungen unterliegen, können durch die Raman-Spektroskopie auch Verunreinigungen wie Verkeimungen, Bakterien oder Viren erkannt werden.

Mit Vorrichtungen und Verfahren nach Ausführungsbeispielen können objektive Aussagen darüber getroffen werden, ob die Blutkonserve 2 für eine Transfusion verwendet werden darf. Die quantitative Auswertung des Raman-Spektrums liefert objektive Information zu den vorhandenen Zellen und/oder dazu, ob ein signifikanter Anteil von Erythrozyten oder Thrombozyten in ihrer Funktion beeinträchtigt ist, beispielsweise durch Zelltod oder andere Prozesse. Die quantitative Auswertung des Raman-Spektrums kann alternativ oder zusätzlich verwendet werden, um Verunreinigungen zu erkennen und so die Sterilität der Blutkonserve zu überprüfen.

Gemäß Erfindung ist die Vorrichtung 1 so eingerichtet, dass Zellen, beispielsweise Erythrozyten oder Thrombozyten, bei der Raman-Spektroskopie in einer optischen Falle gehalten werden. Die optische Falle kann durch den Anregungsstrahl 17 des Raman-Spektroskopiesystems 1 oder einen davon verschiedenen Strahl elektromagnetischer Strahlung erzeugt werden.

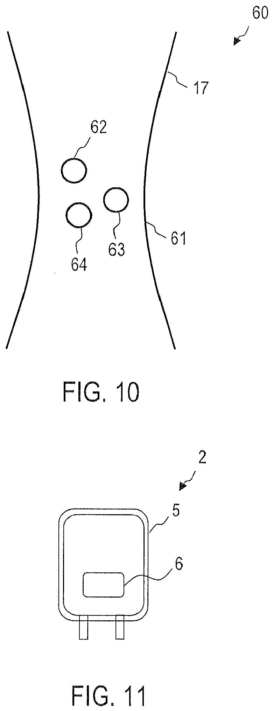

FIG. 10 zeigt beispielhaft eine Konfiguration 60 bei einer Vorrichtung 1 nach einem Ausführungsbeispiel, bei der biologische Objekte in einer optischen Falle gehalten werden, um die Raman-Spektroskopie auszuführen. Ein Fokus 61 eines Strahls erzeugt ein optisches Fallenpotential, in dem biologische Objekte 62-64 zur Raman-Spektroskopie gefangen sind. Der Fokus 61 kann durch den Anregungsstrahl 17 erzeugt werden, der von der Lichtquelle 11 ausgegeben wird. Der Anregungsstrahl 17 kann somit sowohl als Anregung für die Ramanstreuung als auch zur Erzeugung der optischen Falle verwendet werden. Alternativ kann die optische Falle auch von einem separaten Strahl erzeugt werden.

Das Raman-Spektroskopiesystem 10 kann auch einen Lichtleiter, beispielsweise eine optische Faser, umfassen, mit dem der Anregungsstrahl 17 und/oder das Raman-Streulicht geführt wird. Der Lichtleiter kann so positioniert werden, dass der aus ihm austretende Anregungsstrahl die optische Falle mit dem Fokus 61 erzeugt.

Während Ausführungsbeispiele unter Bezugnahme auf die Figuren beschrieben wurden, können Abwandlungen bei weiteren Ausführungsbeispielen realisiert sein. Beispielsweise kann das Raman-Spektrum in einer Vielzahl unterschiedlicher Wellenzahlbereiche oder mit einer Vielzahl unterschiedlicher Methoden der Spektralanalyse ausgewertet werden, um zu bestimmen, ob das Blutprodukt für eine Transfusion verwendet werden darf. Während Ausführungsbeispiele beschrieben wurden, bei denen die Probe durch Entnahme einer Blutproduktprobe aus der Blutkonserve erzeugt wird, kann bei weiteren Ausführungsbeispielen die Raman-Spektroskopie an der Blutkonserve selbst ausgeführt werden.

FIG. 11 veranschaulicht eine Blutkonserve 2 mit einem Beutel 5 aus Kunststoffmaterial. Für eine Verwendung bei Verfahren oder Vorrichtungen nach Ausführungsbeispielen kann der Beutel 5 aus einem Material bestehen, der für den Anregungsstrahl und das Raman-Streulicht der relevanten biologischen Objekte eine hohe Transmissivität aufweist.

Der Beutel 5 kann ein Fenster 6 aus einem Material aufweisen, das für den Anregungsstrahl und das Raman-Streulicht der relevanten biologischen Objekte (beispielsweise Erythrozyten, Thrombozyten, Bakterien und/oder Keime) eine hohe Transmissivität aufweist. Die Vorrichtung 1 kann einen Objektträger aufweisen, der eingerichtet ist, um den Beutel 5 so aufzunehmen, dass das Fenster 6 positioniert ist, um einen Durchtritt des Anregungsstrahls von der Lichtquelle 11 und einen Austritt des Raman-Streulichts zum Raman-Spektrometer 14 zu erlauben.

Die Vorrichtung 1 kann eingerichtet sein, um beispielsweise Ramanpeaks zu identifizieren und auszuwerten, die Erythrozyten oder Thrombozyten zugeordnet sind. Die Vorrichtung 1 kann eingerichtet sein, um beispielsweise Ramanpeaks zu identifizieren und auszuwerten, die Leukozyten zugeordnet sind.

Vorrichtungen und Verfahren nach Ausführungsbeispielen können allgemein zur quantitativen Untersuchung von Blut eingesetzt werden, beispielsweise zur Qualitätskontrolle von Blutkonserven in Blutbanken.

## Patentansprüche

1. Verfahren zur Qualitätskontrolle eines Blutprodukts (2), das ein Erythrozytenkonzentrat, ein Thrombozytenkonzentrat, ein Granulozytenkonzentrat, ein Leukozytenkonzentrat, Vollblut oder Blutplasma umfasst, wobei das Verfahren umfasst:
Bereitstellen eines Computers mit einem oder mehreren Prozessoren oder Controllern,
Hinterlegen von Referenzspektren in einer Datenbank in einem Speicher des Computers, wobei die gespeicherten Referenzspektren eines oder mehrere der folgenden Elemente enthalten:
Lage von Raman-Peaks und/oder Wellenzahlbereichen von frischen Zellen eines oder mehrerer Zelltypen der genannten Blutprodukte;
Lage von Raman-Peaks und/oder Wellenzahlbereichen von funktionell veränderten Zellen eines oder mehrerer Zelltypen der genannten Blutprodukte;
und
Informationen über Bereiche eines mehrdimensionalen Raumes einer Clusteranalyse, in denen die Referenzspektren jeweils angeordnet sind;
Fangen wenigstens einer Zelle einer Probe der genannten Blutprodukte, wobei die Probe Blutzellen enthält, die sich in Lösung befinden, in einer optischen Falle;
Erfassen eines Raman-Spektrums (40; 71, 72) durch Raman-Spektroskopie der wenigstens einen Zelle der Probe (9) des Blutprodukts (2),
Vergleichen des aufgezeichneten Raman-Spektrums der wenigstens einen Zelle der Probe mit den in der Datenbank gespeicherten Referenzspektren,
Auswerten von Zelltod, Sterilität, Verunreinigungen oder funktionellen Veränderungen eines Zelltyps der Probe basierend auf den Ergebnissen des Vergleichs, und
Bestimmen, ob das Blutprodukt (2) für eine Transfusion verwendbar ist, anhand der Auswertung des Raman-Spektrums (40; 71, 72).

2. Verfahren nach Anspruch 1,
wobei durch die Auswertung des Raman-Spektrums (40; 71, 72) quantitativ ermittelt wird, welcher Anteil der Zellen des wenigstens einen Zelltyps einer funktionellen Veränderung unterliegt.

3. Verfahren nach Anspruch 2,
wobei zur quantitativen Ermittlung, welcher Anteil der Zellen des wenigstens einen Zelltyps der funktionellen Veränderung unterliegt, mehrere erfasste Raman-Spektren jeweils einer Hauptkomponentenanalyse unterzogen werden.

4. Verfahren nach einem der vorhergehenden Ansprüche,
wobei durch die Auswertung des Raman-Spektrums (40; 71, 72) eine Anwesenheit von Bakterien oder andere Verunreinigungen in dem Blutprodukt (2) erkannt wird, um zu bestimmen, ob das Blutprodukt (2) für eine Transfusion verwendbar ist.

5. Verfahren nach einem der vorhergehenden Ansprüche,
wobei die Auswertung des Raman-Spektrums (40; 71, 72) eine Spektralanalyse des Raman-Spektrums (40; 71, 72) umfasst.

6. Verfahren nach einem der vorhergehenden Ansprüche,
wobei die optische Falle durch einen Anregungsstrahl (17) eines Raman-Spektroskopiesystems (10) erzeugt wird.

7. Verfahren nach Anspruch 6,
wobei die wenigstens eine Zelle (62-64) ausgewählt ist aus einer Gruppe bestehend aus Erythrozyten, Thrombozyten, Granulozyten und Leukozyten.

8. Vorrichtung zur Qualitätskontrolle eines Blutprodukts (2), das ein Erythrozytenkonzentrat, ein Thrombozytenkonzentrat, ein Granulozytenkonzentrat, ein Leukozytenkonzentrat, Vollblut und/oder Blutplasma umfasst, wobei die Vorrichtung (1) umfasst:
einen Computer mit einem oder mehreren Prozessoren oder Controllern, wobei der Computer eingerichtet ist, um Referenzspektren in einer Datenbank in einem Speicher des Computers zu hinterlegen, wobei die gespeicherten Referenzspektren eines oder mehrere der folgenden Elemente enthalten:
Lage von Raman-Peaks und/oder Wellenzahlbereichen von frischen Zellen eines oder mehrerer Zelltypen der genannten Blutprodukte;
Lage von Raman-Peaks und/oder Wellenzahlbereichen von funktionell veränderten Zellen eines oder mehrerer Zelltypen der genannten Blutprodukte;
und
Informationen über Bereiche eines mehrdimensionalen Raumes einer Clusteranalyse, in denen die Referenzspektren jeweils angeordnet sind;
ein Raman-Spektroskopiesystem (10) zum Erfassen eines Raman-Spektrums (40; 71, 72) einer Probe (9) des Blutprodukts (2),
wobei das Raman-Spektroskopiesystem eingerichtet ist, durch einen Anregungsstrahl (17) oder durch einen von dem Anregungsstrahl (17) verschiedenen Strahl elektromagnetischer Strahlung eine optische Falle zu erzeugen, in der Zellen der Probe (9) der genannten Blutprodukte, wobei die Probe Blutzellen enthält, die sich in Lösung befinden, zur Raman-Spektroskopie gefangen werden,
und ein Spektrum oder mehrere Raman-Spektren zu erfassen und
eine Auswerteeinrichtung (20), die mit dem Raman-Spektroskopiesystem (10) gekoppelt ist und die eingerichtet ist, um das erfasste Raman-Spektrum (40; 71, 72)der wenigstens einen Zelle der Probe mit in der Datenbank gespeicherten Referenzspektren zu vergleichen, um Zelltod, Sterilität, Verunreinigungen oder funktionelle Veränderungen eines Zelltyps der Probe basierend auf den Ergebnissen des Vergleichs auszuwerten und um zu bestimmen, ob das Blutprodukt (2) für eine Transfusion verwendbar ist.

9. Vorrichtung nach Anspruch 8,
wobei die Auswerteeinrichtung (20) zur Ausführung einer Clusteranalyse, insbesondere einer Hauptkomponentenanalyse, eingerichtet ist, um funktionelle Veränderung von Zellen wenigstens eines Zelltyps des Blutprodukts zu erkennen, wobei der Zelltyp ausgewählt ist aus einer Gruppe bestehend aus Erythrozyten, Thrombozyten, Granulozyten und Leukozyten.

10. Vorrichtung nach Anspruch 10,
wobei die Auswerteeinrichtung (20) eingerichtet ist, um durch die Clusteranalyse quantitativ zu ermitteln, welcher Anteil der Zellen des wenigstens einen Zelltyps einer funktionellen Veränderung unterliegt.

11. Vorrichtung nach einem der Ansprüche 8 bis 10, die zur Durchführung des Verfahrens nach einem der Ansprüche 1 bis 7 eingerichtet ist.

## Claims

1. A method for quality control of a blood product (2) comprising an erythrocyte concentrate, a platelet concentrate, a granulocyte concentrate, a leukocyte concentrate, whole blood or blood plasma, the method comprising:
providing a computer having one or more processors or controllers,
storing, in a database in a memory of the computer, reference spectra, wherein the stored reference spectra includes one or more of the following:
position of Raman peaks and/or wavenumber ranges of fresh cells of one or more cell types of said blood products;
position of Raman peaks and/or wavenumber ranges of functionally modified cells of one or more cell types of said blood products; and
information regarding ranges of a multi-dimensional space of a cluster analysis in which the reference spectra are respectively arranged;
trapping at least one cell of a sample of said blood products, said sample containing blood cells that are in solution, in an optical trap;
recording a Raman spectrum (40; 71, 72) by Raman spectroscopy of the at least one cell of the sample (9) of the blood product (2),
comparing the recorded Raman spectrum of the at least one cell of the sample with reference spectra stored in the database,
evaluating cell death, sterility, contaminants, or functional modifications of a cell type of the sample based on the results of the comparison, and
determining whether the blood product (2) is usable for transfusion based on the evaluation of the Raman spectrum (40; 71, 72).

2. The method according to claim 1,
wherein the evaluation of the Raman spectrum (40; 71, 72) quantitatively determines which proportion of the cells of the at least one cell type is subject to a functional modification.

3. The method according to claim 2, wherein for the quantitative determination of which proportion of the cells of the at least one cell type is subject to the functional modification, a plurality of recorded Raman spectra are each subjected to a principal component analysis.

4. The method according to any one of the preceding claims, wherein by evaluating the Raman spectrum (40; 71, 72) a presence of bacteria or other contaminants in the blood product (2) is detected to determine whether the blood product (2) is usable for transfusion.

5. The method according to any one of the preceding claims, wherein the evaluation of the Raman spectrum (40; 71, 72) comprises a spectral analysis of the Raman spectrum (40; 71, 72) .

6. The method according to any one of the preceding claims, wherein the optical trap is generated by an excitation beam (17) of a Raman spectroscopy system (10).

7. The method according to claim 6,
wherein the at least one cell (62-64) is selected from a group consisting of erythrocytes, platelets, granulocytes and leukocytes.

8. A device for quality control of a blood product (2) comprising an erythrocyte concentrate, a platelet concentrate, a granulocyte concentrate, a leukocyte concentrate, whole blood and/or blood plasma, wherein the device (1) comprises:
a computer having one or more processors or controllers, the computer being arranged to store reference spectra in a database in a memory of the computer, wherein the stored reference spectra include one or more of the following:
position of Raman peaks and/or wavenumber ranges of fresh cells of one or more cell types of said blood products;
position of Raman peaks and/or wavenumber ranges of functionally modified cells of one or more cell types of said blood products; and
information regarding ranges of a multi-dimensional space of a cluster analysis in which the reference spectra are respectively arranged;
a Raman spectroscopy system (10) for recording a Raman spectrum (40; 71, 72) of a sample (9) of the blood product (2),
wherein the Raman spectroscopy system is arranged to generate, by an excitation beam (17) or by a beam of electromagnetic radiation different from the excitation beam (17), an optical trap in which cells of the sample (9) of said blood products, the sample containing blood cells which are in solution, are trapped for Raman spectroscopy,
and to record one or more Raman spectra; and
an evaluation device (20) coupled to the Raman spectroscopy system (10) and arranged to compare the recorded Raman spectrum (40; 71, 72)of the at least one cell of the sample with reference spectra stored in the database to evaluate cell death, sterility, contaminants or functional modifications of a cell type of the sample based on the results of the comparison and to determine whether the blood product (2) is usable for transfusion.

9. The device according to claim 8,
wherein the evaluation device (20) is arranged to perform a cluster analysis, in particular a principal component analysis, to detect functional modifications of cells of at least one cell type of the blood product, wherein the cell type is selected from a group consisting of erythrocytes, platelets, granulocytes and leukocytes.

10. The device according to claim 10,
wherein the evaluation device (20) is arranged to quantitatively determine, by the cluster analysis, which proportion of the cells of the at least one cell type is subject to a functional modification.

11. The device according to any one of claims 8 to 10, which is arranged to carry out the method according to any one of claims 1 to 7.

## Revendications

1. Procédé permettant le contrôle de la qualité d'un produit sanguin (2) comprenant un concentré d'érythrocytes, un concentré de plaquettes, un concentré de granulocytes, un concentré de leucocytes, du sang total ou du plasma sanguin, le procédé comprenant :
la fourniture d'un ordinateur comportant un ou plusieurs processeurs ou contrôleurs,
le stockage de spectres de référence dans une base de données dans une mémoire de l'ordinateur, les spectres de référence mémorisés contenant un ou plusieurs des éléments suivants :
position de pics Raman et/ou de plages de nombres d'onde de cellules fraîches d'un ou de plusieurs types de cellules desdits produits sanguins ;
position de pics Raman et/ou de plages de nombres d'onde de cellules modifiées de manière fonctionnelle d'un ou de plusieurs types de cellules desdits produits sanguins ;
et
des informations concernant des zones d'un espace multidimensionnel d'une analyse par grappes, dans lesquelles zones les spectres de référence sont respectivement disposés ;
capture, dans un piège optique, d'au moins une cellule d'un échantillon desdits produits sanguins, l'échantillon contenant des cellules sanguines se trouvant en solution ;
détection d'un spectre Raman (40 ; 71, 72) par spectroscopie Raman de l'au moins une cellule de l'échantillon (9) du produit sanguin (2),
comparaison du spectre Raman enregistré de l'au moins une cellule de l'échantillon avec les spectres de référence mémorisés dans la base de données,
évaluation de la mort cellulaire, de la stérilité, des impuretés ou des modifications fonctionnelles d'un type de cellule de l'échantillon sur la base des résultats de la comparaison, et
détermination du fait de savoir si le produit sanguin (2) peut être utilisé pour une transfusion sur la base de l'évaluation du spectre Raman (40 ; 71, 72).

2. Procédé selon la revendication 1,
l'évaluation du spectre Raman (40 ; 71, 72) permettant de définir quantitativement quelle proportion des cellules de l'au moins un type de cellule subit une modification fonctionnelle.

3. Procédé selon la revendication 2,
pour la détermination quantitative de la proportion des cellules de l'au moins un type de cellule soumise à la modification fonctionnelle, plusieurs spectres Raman détectés étant respectivement soumis à une analyse de composants principaux.

4. Procédé selon l'une des revendications précédentes,
l'évaluation du spectre Raman (40 ; 71, 72) permettant de détecter la présence de bactéries ou d'autres impuretés dans le produit sanguin (2) afin de déterminer si le produit sanguin (2) peut être utilisé pour une transfusion.

5. Procédé selon l'une des revendications précédentes,
l'évaluation du spectre Raman (40 ; 71, 72) comprenant une analyse spectrale du spectre Raman (40 ; 71, 72).

6. Procédé selon l'une des revendications précédentes,
le piège optique étant généré par un faisceau d'excitation (17) d'un système de spectroscopie Raman (10).

7. Procédé selon la revendication 6,
l'au moins une cellule (62-64) étant choisie dans un groupe constitué d'érythrocytes, de plaquettes, de granulocytes et de leucocytes.

8. Dispositif permettant le contrôle de la qualité d'un produit sanguin (2) comprenant un concentré d'érythrocytes, un concentré de plaquettes, un concentré de granulocytes, un concentré de leucocytes, du sang total et/ou du plasma sanguin, le dispositif (1) comprenant :
un ordinateur comportant un ou plusieurs processeurs ou contrôleurs, l'ordinateur étant configuré pour stocker des spectres de référence dans une base de données dans une mémoire de l'ordinateur, les spectres de référence mémorisés contenant un ou plusieurs des éléments suivants :
position de pics Raman et/ou de plages de nombres d'onde de cellules fraîches d'un ou de plusieurs types de cellules desdits produits sanguins ;
position de pics Raman et/ou de plages de nombres d'onde de cellules modifiées de manière fonctionnelle d'un ou de plusieurs types de cellules desdits produits sanguins ;
et
des informations concernant des zones d'un espace multidimensionnel d'une analyse par grappes, dans lesquelles zones les spectres de référence sont respectivement disposés ;
un système de spectroscopie Raman (10) pour la détection d'un spectre Raman (40 ; 71, 72) d'un échantillon (9) du produit sanguin (2),
le système de spectroscopie Raman étant configuré pour générer un piège optique dans les cellules de l'échantillon (9) desdits produits sanguins au moyen d'un faisceau d'excitation (17) ou au moyen d'un faisceau à rayonnement électromagnétique différent du faisceau d'excitation (17), l'échantillon contenant des cellules sanguines se trouvant en solution et capturées par spectroscopie Raman,
et pour détecter un spectre ou plusieurs spectres Raman et
un appareil d'évaluation (20) qui est couplé au système de spectroscopie Raman (10) et configuré pour comparer le spectre Raman (40 ; 71, 72) détecté de l'au moins une cellule de l'échantillon avec des spectres de référence mémorisés dans la base de données afin d'évaluer la mort cellulaire, la stérilité, les impuretés ou les modifications fonctionnelles d'un type de cellule de l'échantillon sur la base des résultats de la comparaison et pour déterminer si le produit sanguin (2) peut être utilisé pour une transfusion.

9. Dispositif selon la revendication 8,
l'appareil d'évaluation (20) étant configuré pour la réalisation d'une analyse par grappes, en particulier une analyse de composants principaux, afin de détecter une modification fonctionnelle de cellules d'au moins un type de cellule du produit sanguin, le type de cellule étant choisi dans le groupe constitué d'érythrocytes, de plaquettes, de granulocytes et de leucocytes.

10. Dispositif selon la revendication 10,
l'appareil d'évaluation (20) étant configuré pour définir quantitativement, au moyen de l'analyse par grappes, quelle proportion des cellules de l'au moins un type de cellule subit une modification fonctionnelle.

11. Dispositif selon l'une des revendications 8 à 10, lequel est configuré pour la mise en oeuvre du procédé selon l'une des revendications 1 à 7.
